# EUROPÄISCHE PATENTSCHRIFT

(11) **EP 0 435 153 B1**
(45) Veröffentlichungstag und Bekanntmachung des Hinweises auf die Patenterteilung: **24.04.1996**
(21) Anmeldenummer: 90124747.8
(22) Anmeldetag: 19.12.1990
(51) Int. Cl.: C07D 473/06, A61K 31/52, C12P 17/18, C12P 41/00

(54) **R-(-)-1-(5-Hydroxyhexyl)-3-methyl-7-propylxanthin, Verfahren zu dessen Herstellung und diese Verbindung enthaltende Arzneimittel**
R-(-)-1-(5-hydroxyhexyl)-3-methyl-7-propylxanthin, process for its preparation and medicines containing it
R-(-)-1-(5-hydroxyhexyl)-3-méthyl-7-propylxanthine, son procédé de préparation et médicaments le contenant

(30) Priorität: 23.12.1989 DE 3942871
(43) Veröffentlichungstag der Anmeldung: 03.07.1991
(73) Patentinhaber: HOECHST AKTIENGESELLSCHAFT, D-65926 Frankfurt am Main (DE)
(72) Erfinder: Furrer, Harald, Dr., W-6238 Hofheim am Taunus (DE); Gebert, Ulrich, Dr., W-6246 Schlossborn (DE); Rudolphi, Karl, Dr., W-6078 Neu-Isenburg (DE)

(56) Entgegenhaltungen:
- EP-A- 0 198 440
- EP-A- 0 272 605
- FR-A- 2 236 501

## Beschreibung

Es ist bekannt, daß die racemische Verbindung 1-(5-Hydroxyhexyl)-3-methyl-7-propylxanthin u. a. eine gehirndurchblutungsfördernde Wirkung besitzt (DE-C-23 66 527). Die Formel der Verbindung ist: 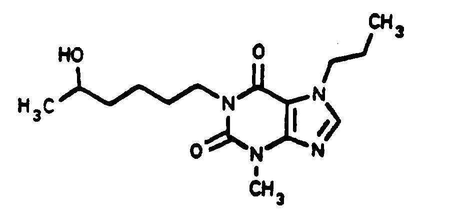 Das die Hydroxylgruppe tragende C-Atom in der Hydroxyhexylseitenkette ist asymmetrisch. Über entsprechende Enantiomere wurde aber noch nicht berichtet.

Wenn man die gehirndurchblutungsfördernde Wirkung des Racemats gleich 1 setzt, kommt der einen der beiden enantiomeren Verbindungen - dem S-(+)-Enantiomeren - ein Wert von etwa 0,65 zu. Für die andere enantiomere Verbindung - das R-(-)-Enantiomere - war daher ein Wert von etwa 1,35 zu erwarten. Überraschenderweise liegt der Wert für das R-(-)-Enantiomere jedoch bei etwa 2. Das bedeutet, daß die Gehirndurchblutungsförderung des R-(-)-Enantiomeren etwa dreimal so stark ist wie diejenige des S-(+)-Antipoden; die Wirkungsdauer der R-(-)-Verbindung beträgt ebenfalls etwa das Dreifache der Wirkungsdauer der S-(+)-Verbindung.

Die R-(-)-Verbindung ist daher Gegenstand der vorliegenden Erfindung.

Die Herstellung der R-(-)-Verbindung kann nach mehreren Methoden erfolgen.

Ein Verfahren besteht beispielsweise in der Einführung des bereits R-konfigurierten 5-Hydroxyhexylrestes in die 1-Position von 3-Methyl-7-propylxanthin nach bekannten Verfahrensweisen;
aber auch eine Auftrennung des Racemats in die Antipoden nach bekannten Standardmethoden ist möglich.

Eine weitere Herstellungsmöglichkeit ist die stereoselektive Synthese aus 1-(5-Oxohexyl)-3-methyl-7-propylxanthin; dabei ist die stereoselektive Reduktion mit Hilfe von Mikroorganismen bevorzugt.

Ein derartiges Verfahren wir din der deutschen Patentanmeldung P 39 42 872.9 beschrieben. Dieses Verfahren besteht darin, daß man 1-(5-Oxohexyl)-3-methyl-7-propylxanthin mit dem Hefestamm Rhodotorula rubra (der bei der Deutschen Sammlung von Mikroorganismen und Zellkulturen am 10.7.1989 unter der Nummer DSM 5436 hinterlegt wurde) stereospezifisch reduziert, wobei S-(+)-1-(5-Hydroxyhexyl)-3-methyl-7-propyl-xanthin entsteht, das dann einer Konfigurationsumkehr unterworfen wird.

Nach einem bevorzugten anderen mikrobiellen Verfahren wird
a) 1-(5-Oxohexyl)-3-methyl-7-propylxanthin mit Bäckerhefe (Saccharomyces cerevisiae; Hersteller: Deutsche Hefewerke GmbH, Frankfurt/Main) stereoselektiv zu S-(+)-1-(5-Hydroxyhexyl)-3-methyl-7-propyl-xanthin reduziert, und
b) dieses durch Konfigurationsumkehr in R-(-)-1-(5-Hyroxyhexyl-3-methyl-7-propylxanthin überführt.

Die Reduktion mit Bäckerhefe wird zweckmäßig zwischen Raumtemperatur und etwa 40°C, bevorzugt in Gegenwart von Saccharose, im allgemeinen bei pH-Werten von etwa 7 bis 8,5 in Wasser oder in Gemischen von Wasser mit wassermischbaren - hier inerten - organischen Lösungsmitteln wie Ethanol, Ethylenglycol oder Dimethylformamid durchgeführt, wobei die Reaktionszeit von wenigen Stunden bis zu mehreren Tagen reichen kann.

Die nachfolgende Konfigurationsumkehr des S-(+)- zu dem R-(-)-Enantiomeren wird vorteilhaft mit einem tertiären Phosphin (bevorzugt Triphenylphosphin),
einer organischen Carbonsäure (bevorzugt Benzoesäure) und einem Azodicarbonsäuredialkylester (bevorzugt Azodicarbonsäurediethylester)
in einem aprotischen Lösungsmittel (bevorzugt in Tetrahydrofuran)
im allgemeinen bei Temperaturen zwischen etwa 20 bis etwa 30°C durchgeführt,
und der dabei gebildete Carbonsäureester des R-(-)-Enantiomeren wird danach durch Solvolyse nach bekannten Verfahren in alkoholischen oder wäßrigen Lösungsmitteln in Gegenwart von basischen Stoffen, insbesondere durch Methanolyse in Anwesenheit von Kaliumcarbonat, gespalten. Das Solvolyseprodukt wird dann in bekannter Weise aufgearbeitet.

Die Konfigurationsumkehr kann in ähnlich vorteilhafter Weise auch so erfolgen, daß man das S-(+)-Enantiomer
(1) mit einem Sulfonsäurehalogenid, gegebenenfalls in Gegenwart einer Base, in einem aprotischen Lösungsmittel in den entsprechenden Sulfonsäureester überführt,
(2) diesen mit einem Alkalisalz einer aliphatischen Carbonsäure in einem aprotischen Lösungsmittel zu dem entsprechenden Carbonsäureester umsetzt, wobei die Konfigurationsumkehr stattfindet, und
(3) aus diesem Carbonsäureester durch Solvolyse in einem alkoholischen oder wäßrigen Lösungsmittel in Gegenwart eines basischen Stoffes die Verbindung gemäß Anspruch 1 freisetzt.

In Stufe (1) ist bevorzugt, als organisches Sulfonsäurehalogenid Methansulfonsäurechlorid oder p-Toluolsulfonsäurechlorid, als Base Triethylamin und als aprotisches Lösungsmittel Pyridin und/oder Dichlormethan einzusetzen;
bevorzugtes Alkalisalz einer aliphatischen Carbonsäure - in Stufe (2) - ist Cäsiumpropionat und bevorzugtes aprotisches Lösungsmittel in dieser Stufe Dimethylformamid und/oder Dimethylsulfoxid. Diese Stufe wird im allgemeinen bei Temperaturen zwischen etwa 20 und 100°C in wenigen Stunden bis zu mehreren Tagen durchgeführt.
Als Lösungsmittel für die Solvolyse - Stufe (3) wird bevorzugt Methanol und als bevorzugter basischer Stoff in dieser Stufe Kaliumcarbonat verwendet.

Erfindungsgegenstand sind weiterhin Arzneimittel, welche durch einen Gehalt an R-(-)-1-(5-Hydroxyhexyl)-3-methyl-7-propylxanthin gekennzeichnet sind.

Die Arzneimittel eignen sich insbesondere zur Prophylaxe und Therapie von cerebralen Gefäßerkrankungen.

Die erfindungsgemäßen Arzneimittel können oral, rektal oder parenteral verabreicht werden.

Geeignete feste oder flüssige galenische Zubereitungsformen sind beispielsweise Granulate, Pulver, Tabletten, Dragees, (Mikro)Kapseln, Zäpfchen, Sirupe, Emulsionen, Suspensionen, Tropfen oder injizierbare Lösungen sowie Präparate mit protrahierter Wirkstoff-Freigabe, bei deren Herstellung üblicherweise Hilfsmittel, wie Trägerstoffe, Spreng-, Binde-, Überzugs-, Quellungs-, Gleit- oder Schmiermittel, Geschmacksstoffe, Süßungsmittel oder Lösungsvermittler, Verwendung finden. Als häufig verwendete Hilfsstoffe seien z. B. Laktose, Mannit und andere Zucker, Talkum, Milcheiweiß, Gelatine, Stärke, Cellulose und ihre Derivate, tierische und pflanzliche Öle, Polyethylenglykole und Lösungsmittel, wie etwa steriles Wasser, genannt.

Das nachfolgende Beispiel soll die Herstellung der erfindungsgemäßen Verbindung illustrieren; danach folgt dann noch ein Versuchsbericht, aus welchem die überraschend erhöhte gehirndurchblutungsfördernde Wirkung und die günstige Wirkungsdauer dieser Verbindung hervorgehen.

### Beispiel:

### a)Mikrobielle Reduktion von 1-(5-Oxohexyl)-3-methyl-7-propyl-xanthin zu S-(+)-1-(5-Hydroxyhexyl)-3-methyl-7-propyl-xanthin

1200 g Saccharose und 840 g Bäckerhefe (Goldhefe, Deutsche Hefewerke GmbH) in 6.5 1 Wasser werden bei 30-35°C gerührt. Nach 1 h tropft man innerhalb von 10 Minuten 184 g 1-(5-Oxohexyl)-3-methyl-7-propylxanthin hinzu , versetzt nach 24 Stunden wiederum mit 1200 g Saccharose und 840 g Bäckerhefe in 5.5 1 Wasser, nach weiteren 24 Stunden nochmals mit 800 g Saccharose und rührt noch 8 Stunden.

In die Suspension rührt man 1.8 kg Celite ein, filtriert über eine Drucknutsche und wäscht mit Isopropanol nach. Die Filtrate werden vereinigt, unter vermindertem Druck auf ca. 2 1 eingeengt und mit Dichlormethan und Wasser extraktiv aufgearbeitet. Die Dichlormethanphase trocknet man und engt unter vermindertem Druck ein. Der Rückstand wird durch Säulenchromatographie an Kieselgel (Laufmittel: Dichlormethan/Ethanol, Volumenverhältnis: 98/2) und durch Kugelrohrdestillation bei 0.1 mbar und 140-145°C Badtemperatur gereinigt.

- Ausbeute:: 21.7 g
- Schmelzpunkt:: 81-82°C
- Enantiomerenreinheit:: > 95 %
[α]_{D} ²⁰ = + 4.6° (c = 6.7, C₂H₅OH)

| Analyse: | | | |
|---|---|---|---|
| ber. : | C 58.42 % | H 7.84 % | N 18.17 % |
| gef. : | C 58.50 % | H 7.95 % | N 18.19 % |

### b) Konfigurationsumkehr des S-(+)- zu R-(-)-1-(5-Hydroxyhexyl)-3-methyl-7-propyl-xanthin

Zum Gemisch aus 18.5 g S-(+)-1-(5-Hydroxyhexyl)-3-methyl-7-propylxanthin, 7.3 g Benzoesäure und 16.1 g Triphenylphosphin in 60 ml wasserfreiem Tetrahydrofuran tropft man bei 20-25°C die Lösung von 10.9 g Azodicarbonsäurediethylester in 60 ml wasserfreiem Tetrahydrofuran.

Nach 24 h Rühren bei 20-25°C engt man unter vermindertem Druck ein, reinigt den Rückstand durch Säulenchromatographie an Kieselgel (Laufmittel: Dichlormethan/Ethanol, Volumenverhältnis: 95/5) und erhält 50.5 g rohes R-1-(5-Benzoyloxy)-3-methyl-7-propyl-xanthin. Dieses erhitzt man in 750 ml Methanol zusammen mit 4 g Kaliumcarbonat 32 h bei Rückflußtemperatur und engt bei vermindertem Druck ein. Nach extraktiver Aufarbeitung des Rückstandes mit Dichlormethan und Wasser erhält man nach Einengen der getrockneten Dichlormethanphase 48.5 g Rohprodukt, das man durch Säulenchromatographie an Kieselgel (Laufmittel: Dichlormethan/Ethanol, Volumenverhältnis: 95/5 -> 90/10) und Kugelrohrdestillation bei 140°C Badtemperatur und 0.1 mbar reinigt.
- Ausbeute:: 13.9 g
- Schmelzpunkt:: 81-82°C
[α]_{D} ²⁰ = - 4.5° (c = 6.4, C₂H₅OH)
- Enantiomerenreinheit:: > 95 %

| Analyse: | | | |
|---|---|---|---|
| ber. : | C 58.42 % | H 7.84 % | N 18.17 % |
| gef. : | C 58.35 % | H 8.05 % | N 18.36 % |

Die Struktur der Verbindungen wurde durch Elementaranalyse und IR- sowie ¹H-NMR-Spektren gesichert. Die Bestimmung von absoluter Konfiguration und Enantiomerenreinheit erfolgte über die Mosher-Ester mit S-(-)-Methoxy-trifluormethylphenylessigsäure(¹H- bzw. ¹⁹F- NMR-Spektren). Die Enantiomerenreinheit wurde zusätzlich noch durch Gaschromatographie nach Derivatisierung mit S-(-)-1-Phenylethylisocyanat ermittelt.

### Versuchsbericht:

### Hirndurchblutung der Katze

Die Wirkung der erfindungsgemäßen Verbindung 1 auf die regionale Gehirndurchblutung wurde im Vergleich mit dem Racemat 2 und dem S-(+)-Enantiomer 3 mit Hilfe der Wärmeleittechnik nach F. A. Gibbs (Proc. Soc. exp. Biol. (N. Y. ) 31 (1933), S 141 ff), H. Hensel (Naturwissenschaften 43 (1956), S 477 ff) und E. Betz (Acta Neurol. Scand. Suppl. 14 (1965), S. 29-37) an Katzen beiderlei Geschlechts in Natrium-Pentobarbital-Narkose (35 mg/kg i. p. ) untersucht. Bei dieser Methode bestimmt man mit Hilfe einer auf die Hirnoberfläche im Bereich des Gyrus marginalis frontalis aufgesetzten Wärmeleitsonde den Wärmetransport von einem Heizpunkt zu einer benachbarten Temperaturmeßstelle in der Sonde, der der Höhe der Gehirndurchblutung direkt proportional ist. Als Maß für die Durchblutungszunahme dient die mittlere prozentuale Änderung der Wärmetransportzahl λ nach Präparatgabe.

Die Verbindungen wurden als Suspension in 1 %-iger wässriger Carboxymethylcellulose intraduodenal appliziert. Die Dosis betrug 25 mg Testsubstanz pro kg Körpergewicht. Für jedes Prüfpräparat wurden 6 bis 11 Einzelversuche durchgeführt und aus den gewonnen Meßdaten die mittlere prozentuale Änderung der Wärmetransportzahl (Δλ%) ermittelt.

In der Tabelle 1 sind für R-(-)-1-(5-Hydroxyhexyl)-3-methyl-7-propylxanthin (1), das entsprechende Enantiomer S-(+)-1-(5-Hydroxyhexyl)-3-methyl-7-propylxanthin (3) und das Racemat (2) beider Verbindungen die Schmelzpunkte F. , die Enantiomerenreinheiten e.e. (enantiomeric excess), die Anzahl der Messungen n ,die mittlere prozentuale Änderung (Δλ %) der Wärmetransportzahl als Maß für die Wirkungsstärke, die Umrechnungsfaktoren f, bezogen auf Δλ % = 1 für das Racemat und die Wirkungsdauer als Halbwertszeit HWZ (min) angegeben.

**Tabelle 1**

| Änderung der Gehirndurchblutung | | | | | |
|---|---|---|---|---|---|
| Verbindung | F. | e.e. | n | Δλ %(f) | HWZ(min) |
| 1(R-(-)) | 81-82°C | > 95 % | 6 | +45 (1.88) | 100 |
| 2(Rac.) | 76-77°C | - | 7 | +24 (1.00) | 100 |
| 3(S-(+)) | 81-82°C | > 95 % | 11 | +16 (0.67) | 36 |

## Patentansprüche (Patentansprüche für folgende(n) Vertragsstaat(en): AT, BE, CH, DE, DK, FR, GB, IT, LI, LU, NL, SE)

1. R-(-)-1-(5-Hydroxyhexyl)-3-methyl-7-propylxanthin.

2. Verfahren zur Herstellung von R-(-)-1-(5-Hydroxyhexyl)-3-methyl-7-propylxanthin, dadurch gekennzeichnet, daß man
a) 1-(5-Oxohexyl)-3-methyl-7-propylxanthin mit Bäckerhefe (Saccharomyces cerevisiae) zu S-(+)-1-(5-Hydroxyhexyl)-3-methyl-7-propylxanthin reduziert und
b) dieses durch Konfigurationsumkehr in R-(-)-1-(5-Hydroxyhexyl)-3-methyl-7-propylxanthin überführt.

3. Verfahren nach Anspruch 2, dadurch gekennzeichnet, daß man die Konfigurationsumkehr durch Reaktion mit einem tertiären Phosphin, einer Carbonsäure und einem Azodicarbonsäuredialkylester in einem aprotischen Lösungsmittel durchführt,
das Reaktionsprodukt, welches das R-(-)-Enantiomere in Form des Esters mit der eingesetzten Carbonsäure enthält, einer Solvolyse unterwirft
und das Solvolyseprodukt in bekannter Weise aufarbeitet.

4. Verfahren nach Anspruch 3, dadurch gekennzeichnet, daß man als tertiäres Phosphin Triphenylphosphin,
als Carbonsäure Benzoesäure,
als Azodicarbonsäuredialkylester Azodicarbonsäurediethylester
und als aprotisches Lösungsmittel Tetrahydrofuran verwendet,
und daß man die Solvolyse in Form einer Methanolyse in Gegenwart von Kaliumcarbonat durchführt.

5. Verfahren nach Anspruch 2, dadurch gekennzeichnet, daß man zur Konfigurationsumkehr das S-(+)-Enantiomer
(1) mit einem organischen Sulfonsäurehalogenid, gegebenenfalls in Gegenwart einer Base, in einem aprotischen Lösungsmittel in den entsprechenden Sulfonsäureester überführt,
(2) diesen mit einem Alkalisalz einer aliphatischen Carbonsäure in einem aprotischen Lösungsmittel zu dem entsprechenden Carbonsäureester umsetzt, wobei die Konfigurationsumkehr stattfindet, und
(3) aus diesem Carbonsäureester durch Solvolyse in einem alkoholischen oder wäßrigen Lösungsmittel in Gegenwart eines basischen Stoffes die Verbindung gemäß Anspruch 1 freisetzt.

6. Verfahren nach Anspruch 5, dadurch gekennzeichnet, daß man
in Stufe (1) als organisches Sulfonsäurehalogenid Methansulfonsäurechlorid oder p-Toluolsulfonsäurechlorid,
als Base Triethylamin
und als aprotisches Lösungsmittel Pyridin und/oder Dichlormethan,
in Stufe (2) als Alkalisalz einer aliphatischen Carbonsäure Cäsiumpropionat,
als aprotisches Lösungsmittel Dimethylformamid und/oder Dimethylsulfoxid
und in Stufe (3) als Lösungsmittel Methanol sowie als basischen Stoff Kaliumcarbonat verwendet.

7. Arzneimittel, gekennzeichnet durch einen Gehalt an R-(-)-1-(5-Hydroxyhexyl)-3-methyl-7-propylxanthin.

8. Arzneimittel nach Anspruch 7, dadurch gekennzeichnet, daß sie zur Prophylaxe und Therapie von cerebralen Gefäßerkrankungen bestimmt sind.

9. Verwendung von R-(-)-1-(5-Hydroxyhexyl)-3-methyl-7-propylxanthin zur Herstellung eines Arzneimittels zur Prophylaxe und Therapie von cerebralen Gefäßerkrankungen.

## Patentansprüche (Patentansprüche für folgende(n) Vertragsstaat(en): ES, GR)

1. Verfahren zur Herstellung der Verbindung R-(-)-1-(5-Hydroxyhexyl)-3-methyl-7-propylxanthin,
dadurch gekennzeichnet, daß man
a) 1- (5-Oxohexyl)-3-methyl-7-propylxanthin mit Bäckerhefe (Saccharomyces cerevisiae) zu S-(+)-1-(5-Hydroxyhexyl)-3-methyl-7-propylxanthin reduziert und
b) dieses durch Konfigurationsumkehr in R-(-)-1-(5-Hydroxyhexyl)-3-methyl-7-propyl-xanthin überführt.

2. Verfahren nach Anspruch 1, dadurch gekennzeichnet, daß man die Konfigurationsumkehr durch Reaktion mit einem tertiären Phosphin, einer Carbonsäure und einem Azodicarbonsäuredialkylester in einem aprotischen Lösungsmittel durchführt,
das Reaktionsprodukt, welches das R-(-)-Enantiomere in Form des Esters mit der eingesetzten Carbonsäure enthält, einer Solvolyse unterwirft
und das Solvolyseprodukt in bekannter Weise aufarbeitet.

3. Verfahren nach Anspruch 2, dadurch gekennzeichnet, daß man als tertiäres Phosphin Triphenylphosphin,
als Carbonsäure Benzoesäure,
als Azodicarbonsäuredialkylester Azodicarbonsäurediethylester
und als aprotisches Lösungsmittel Tetrahydrofuran verwendet,
und daß man die Solvolyse in Form einer Methanolyse in Gegenwart von Kaliumcarbonat durchführt.

4. Verfahren nach Anspruch 1, dadurch gekennzeichnet, daß man zur Konfigurationsumkehr das S-(+)-Enantiomer
(1) mit einem organischen Sulfonsäurehalogenid, gegebenenfalls in Gegenwart einer Base, in einem aprotischen Lösungsmittel in den entsprechenden Sulfonsäureester überführt,
(2) diesen mit einem Alkalisalz einer aliphatischen Carbonsäure in einem aprotischen Lösungsmittel zu dem entsprechenden Carbonsäureester umsetzt, wobei die Konfigurationsumkehr stattfindet, und
(3) aus diesem Carbonsäureester durch Solvolyse in einem alkoholischen oder wäßrigen Lösungsmittel in Gegenwart eines basischen Stoffes die Verbindung gemäß Anspruch 1 freisetzt.

5. Verfahren nach Anspruch 4, dadurch gekennzeichnet, daß man
in Stufe (1) als organisches Sulfonsäurehalogenid Methansulfonsäurechlorid oder p-Toluolsulfonsäurechlorid,
als Base Triethylamin
und als aprotisches Lösungsmittel Pyridin und/oder Dichlormethan,
in Stufe (2) als Alkalisalz einer aliphatischen Carbonsäure Cäsiumpropionat,
als aprotisches Lösungsmittel Dimethylformamid und/oder Dimethylsulfoxid
und in Stufe (3) als Lösungsmittel Methanol sowie als basischen Stoff Kaliumcarbonat verwendet.

6. Verwendung von R-(-)-1-(5-Hydroxyhexyl)-3-methyl-7-propylxanthin zur Herstellung eines Arzneimittels zur Prophylaxe und Therapie von cerebralen Gefäßerkrankungen.

## Claims (Claims for the following Contracting State(s): AT, BE, CH, DE, DK, FR, GB, IT, LI, LU, NL, SE)

1. R-(-)-1-(5-Hydroxyhexyl)-3-methyl-7-propylxanthine.

2. A process for the preparation of R-(-)-1-(5-hydroxyhexyl)-3-methyl-7-propylxanthine, which comprises
a) reducing 1-(5-oxohexyl)-3-methyl-7-propylxanthine to S-(+)-1-(5-hydroxyhexyl)-3-methyl-7-propylxanthine using baker's yeast (Saccharomyces cerevisiae) and
b) converting this by inversion of configuration into R-(-)-1-(5-hydroxyhexyl)-3-methyl-7-propylxanthine.

3. The process as claimed in claim 2, wherein the inversion of configuration is carried out by reaction with a tertiary phosphine, a carboxylic acid and a dialkyl azodicarboxylate in an aprotic solvent,
the reaction product which contains the R-(-)-enantiomer in the form of the ester with the carboxylic acid employed is subjected to a solvolysis and the solvolysis product is worked up in a known manner.

4. The process as claimed in claim 3, wherein triphenylphosphine is used as the tertiary phosphine, benzoic acid is used as the carboxylic acid, diethyl azodicarboxylate is used as the dialkyl azodicarboxylate
and tetrahydrofuran is used as the aprotic solvent, and wherein the solvolysis is carried out in the form of a methanolysis in the presence of potassium carbonate.

5. The process as claimed in claim 2, wherein, for the inversion of configuration, the S-(+)-enantiomer
(1) is converted into the corresponding sulfonic acid ester using an organic sulfonyl halide, if appropriate in the presence of a base, in an aprotic solvent,
(2) this ester is reacted with an alkali metal salt of an aliphatic carboxylic acid in an aprotic solvent to give the corresponding carboxylic acid ester, whereupon the inversion of configuration takes place, and
(3) the compound as claimed in claim 1 is liberated from this carboxylic acid ester by solvolysis in an alcoholic or aqueous solvent in the presence of a basic substance.

6. The process as claimed in claim 5, wherein,
in step (1), methanesulfonyl chloride or p-toluenesulfonyl chloride is used as the organic sulfonyl halide,
triethylamine is used as the base
and pyridine and/or dichloromethane is used as the aprotic solvent,
in step (2), cesium propionate is used as the alkali metal salt of an aliphatic carboxylic acid,
dimethylformamide and/or dimethyl sulfoxide is used as the aprotic solvent
and, in step (3), methanol is used as the solvent and potassium carbonate is used as the basic substance.

7. A pharmaceutical which contains R-(-)-1-(5-hydroxyhexyl)-3-methyl-7-propylxanthine.

8. A pharmaceutical as claimed in claim 7, which is intended for the prophylaxis and treatment of cerebral vascular disorders.

9. The use of R-(-)-1-(5-hydroxyhexyl)-3-methyl-7-propylxanthine for the preparation of a pharmaceutical for the prophylaxis and treatment of cerebral vascular disorders.

## Claims (Claims for the following Contracting State(s): ES, GR)

1. A process for the preparation of the compound R-(-)-1-(5-hydroxyhexyl)-3-methyl-7-propylxanthine, which comprises
a) reducing 1-(5-oxohexyl)-3-methyl-7-propylxanthine to S-(+)-1-(5-hydroxyhexyl)-3-methyl-7-propylxanthine using baker's yeast (Saccharomyces cerevisiae) and
b) converting this by inversion of configuration into R-(-)-1-(5-hydroxyhexyl)-3-methyl-7-propylxanthine.

2. The process as claimed in claim 1, wherein the inversion of configuration is carried out by reaction with a tertiary phosphine, a carboxylic acid and a dialkyl azodicarboxylate in an aprotic solvent,
the reaction product which contains the R-(-)-enantiomer in the form of the ester with the carboxylic acid employed is subjected to a solvolysis and the solvolysis product is worked up in a known manner.

3. The process as claimed in claim 2, wherein triphenylphosphine is used as the tertiary phosphine, benzoic acid is used as the carboxylic acid, diethyl azodicarboxylate is used as the dialkyl azodicarboxylate
and tetrahydrofuran is used as the aprotic solvent, and wherein the solvolysis is carried out in the form of a methanolysis in the presence of potassium carbonate.

4. The process as claimed in claim 1, wherein, for the inversion of configuration, the S-(+)-enantiomer
(1) is converted into the corresponding sulfonic acid ester using an organic sulfonyl halide, if appropriate in the presence of a base, in an aprotic solvent,
(2) this ester is reacted with an alkali metal salt of an aliphatic carboxylic acid in an aprotic solvent to give the corresponding carboxylic acid ester, whereupon the inversion of configuration takes place, and
(3) the compound as claimed in claim 1 is liberated from this carboxylic acid ester by solvolysis in an alcoholic or aqueous solvent in the presence of a basic substance.

5. The process as claimed in claim 4, wherein,
in step (1), methanesulfonyl chloride or p-toluenesulfonyl chloride is used as the organic sulfonyl halide,
triethylamine is used as the base
and pyridine and/or dichloromethane is used as the aprotic solvent,
in step (2), cesium propionate is used as the alkali metal salt of an aliphatic carboxylic acid,
dimethylformamide and/or dimethyl sulfoxide is used as the aprotic solvent
and, in step (3), methanol is used as the solvent and potassium carbonate is used as the basic substance.

6. The use of R-(-)-1-(5-hydroxyhexyl)-3-methyl-7-propylxanthine for the preparation of a pharmaceutical for the prophylaxis and treatment of cerebral vascular disorders.

## Revendications (Revendications pour l'(les) Etat(s) contractant(s) suivant(s): AT, BE, CH, DE, DK, FR, GB, IT, LI, LU, NL, SE)

1. R-(-)-1-(5-hydroxyhexyl)-3-méthyl-7-propylxanthine.

2. Procédé pour l'élaboration de la R-(-)-1-(5-hydroxyhexyl)-3-méthyl-7-propylxanthine caractérisé en ce que,
a) l'on réduit la 1-(5-oxohexyl)-3-méthyl-7-propylxanthine avec de la levure de boulanger (saccharomyces cerevisiae) en S-(+)-1-(5-hydroxyhexyl)-3-méthyl-7-propylxanthine et en ce que,
b) l'on transforme celle-ci par inversion de configuration en R-(-)-1-(5-hydroxyhexyl)-3-méthyl-7-propylxanthine.

3. Procédé selon la revendication 2, caractérisé en ce que l'on effectue l'inversion de configuration par réaction avec une phosphine tertiaire, un acide carboxylique et un dialkylester d'acide azodicarboxylique au sein d'un solvant aprotique,
en ce que l'on soumet le produit de la réaction à une solvolyse, lequel produit contient le R-(-)-énantiomère sous la forme de l'ester obtenu avec l'acide carboxylique mis en oeuvre et
en ce que l'on traite le produit de la solvolyse par une méthode connue.

4. Procédé selon la revendication 3, caractérisé en ce que l'on utilise la triphénylphosphine comme phosphine tertiaire,
l'acide benzoïque comme acide carboxylique,
le diéthylester d'acide azodicarboxylique comme dialkylester d'acide azodicarboxylique et le tétrahydrofurane comme solvant aprotique,
et en ce que l'on effectue la solvolyse sous la forme d'une méthanolyse en présence de carbonate de potassium.

5. Procédé selon la revendication 2, caractérisé en ce que l'on transforme, pour l'inversion de configuration, le S-(+)-énantiomère
(1) en l'ester sulfonique correspondant en utilisant un halogénure sulfonique organique, le cas échéant, en présence d'une base au sein d'un solvant aprotique, en ce que
(2) l'on transforme celui-ci en l'ester d'acide carboxylique correspondant à l'aide d'un sel alcalin d'un acide carboxylique aliphatique au sein d'un solvant aprotique, à l'occasion de quoi l'inversion de configuration a lieu, et en ce que
(3) l'on récupère par solvolyse à partir de cet ester d'acide carboxylique le composé selon la revendication 1 dans un solvant alcoolique ou aqueux en présence d'une substance basique.

6. Procédé selon la revendication 5, caractérisé en ce que l'on utilise
dans l'étape (1), le chlorure méthane sulfonique ou le chlorure p-toluène sulfonique comme halogénure sulfonique organique,
la triéthylamine comme base et
la pyridine et/ou le dichlorométhane comme solvant aprotique,
dans l'étape (2), le propionate de césium comme sel alcalin d'un acide carboxylique aliphatique,
le diméthylformamide et/ou le diméthylsulfoxyde comme solvant aprotique et
dans l'étape (3), le méthanol comme solvant ainsi que le carbonate de potassium comme substance basique.

7. Médicament caractérisé en ce qu'il contient de la R-(-)-1-(5-hydroxyhexyl)-3-méthyl-7-propylxanthine.

8. Médicament selon la revendication 7, caractérisé en ce qu'il convient à la prophylaxie et à la thérapie des maladies vasculaires cérébrales.

9. Emploi de la R-(-)-1-(5-hydroxyhexyl)-3-méthyl-7-propylxanthine pour l'élaboration d'un médicament utilisé dans la prophylaxie et la thérapie des maladies vasculaires cérébrales.

## Revendications (Revendications pour l'(les) Etat(s) contractant(s) suivant(s): ES, GR)

1. Procédé pour l'élaboration de la R-(-)-1-(5-hydroxyhexyl)-3-méthyl-7-propylxanthine caractérisé en ce que,
a) l'on réduit la 1-(5-oxohexyl)-3-méthyl-7-propylxanthine avec de la levure de boulanger (saccharomyces cerevisiae) en S-(+)-1-(5-hydroxyhexyl)-3-méthyl-7-propylxanthine et en ce que,
b) l'on transforme celle-ci par inversion de configuration en R-(-)-1-(5-hydroxyhexyl)-3-méthyl-7-propylxanthine.

2. Procédé selon la revendication 1, caractérisé en ce que l'on effectue l'inversion de configuration par réaction avec une phosphine tertiaire, un acide carboxylique et un dialkylester d'acide azodicarboxylique au sein d'un solvant aprotique,
en ce que l'on soumet le produit de la réaction à une solvolyse, lequel produit contient le R-(-)-énantiomère sous la forme de l'ester obtenu avec l'acide carboxylique mis en oeuvre et
en ce que l'on traite le produit de la solvolyse par une méthode connue.

3. Procédé selon la revendication 2, caractérisé en ce que l'on utilise la triphénylphosphine comme phosphine tertiaire,
l'acide benzoïque comme acide carboxylique,
le diéthylester d'acide azodicarboxylique comme dialkylester d'acide azodicarboxylique et le tétrahydrofurane comme solvant aprotique,
et en ce que l'on effectue la solvolyse sous la forme d'une méthanolyse en présence de carbonate de potassium.

4. Procédé selon la revendication 1, caractérisé en ce que l'on transforme, pour l'inversion de configuration, le S-(+)-énantiomère
(1) en l'ester sulfonique correspondant en utilisant un halogénure sulfonique organique, le cas échéant, en présence d'une base au sein d'un solvant aprotique, en ce que
(2) l'on transforme celui-ci en l'ester d'acide carboxylique correspondant à l'aide d'un sel alcalin d'un acide carboxylique aliphatique au sein d'un solvant aprotique, à l'occasion de quoi l'inversion de configuration a lieu, et en ce que
(3) l'on récupère par solvolyse à partir de cet ester d'acide carboxylique le composé selon la revendication 1 dans un solvant alcoolique ou aqueux en présence d'une substance basique.

5. Procédé selon la revendication 4, caractérisé en ce que l'on utilise
dans l'étape (1), le chlorure méthane sulfonique ou le chlorure p-toluène sulfonique comme halogénure sulfonique organique,
la triéthylamine comme base et
la pyridine et/ou le dichlorométhane comme solvant aprotique,
dans l'étape (2), le propionate de césium comme sel alcalin d'un acide carboxylique aliphatique,
le diméthylformamide et/ou le diméthylsulfoxyde comme solvant aprotique et
dans l'étape (3), le méthanol comme solvant ainsi que le carbonate de potassium comme substance basique.

6. Emploi de la R-(-)-1-(5-hydroxyhexyl)-3-méthyl-7-propylxanthine pour l'élaboration d'un médicament utilisé dans la prophylaxie et la thérapie des maladies vasculaires cérébrales.
